# EUROPÄISCHE PATENTSCHRIFT

(11) **EP 2 898 943 B1**
(45) Veröffentlichungstag und Bekanntmachung des Hinweises auf die Patenterteilung: **16.11.2016**
(21) Anmeldenummer: 14000295.7
(22) Anmeldetag: 28.01.2014
(51) Int. Cl.: B01J 3/00, C07C 41/01

(54) **Verfahren und Anlage zur Gewinnung von Dimethylether aus Synthesegas**
Process and apparatus for the obtention of dimethylether from syngas
Porcédé et dispositif pur l'obtention du dimethyléther à partir du gaz de synthèse

(43) Veröffentlichungstag der Anmeldung: 29.07.2015
(73) Patentinhaber: Linde Aktiengesellschaft, 80331 München (DE)
(72) Erfinder: Fritz, Helmut, 81375 München (DE); Bartesch, Thomas, 85625 Glonn (DE); Delhomme, Clara, Dr., 81829 München (DE); Peschel, Andreas, Dr., 82515 Wolfratshausen (DE); Klein, Harald, 82515 Wolfratshausen (DE); Fendt, Johannes, 81377 München (DE)

(56) Entgegenhaltungen:
- EP-A1- 1 026 141
- US-A1- 2013 030 063
- "DME - THE NEW WONDER FUEL?", NITROGEN AND METHANOL, BRITISH SULPHUR PUBLISHING, LONDON, GB, Nr. 260, 1. November 2002 (2002-11-01), Seiten 25-31, XP001142297, ISSN: 1462-2378

## Beschreibung

Die Erfindung betrifft ein Verfahren und eine Anlage zur Gewinnung von Dimethylether aus Synthesegas gemäß den Oberbegriffen der unabhängigen Patentansprüche.

### Stand der Technik

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden.

Dimethylether kann entweder direkt aus Methanol oder indirekt aus Erd- oder Biogas erzeugt werden. Im letzteren Fall wird das Erd- oder Biogas zunächst zu Synthesegas reformiert. Synthesegas kann auch mittels anderer Verfahren, beispielsweise durch Pyrolyse von Abfällen oder Biomasse, gewonnen werden. Das Synthesegas wird klassischerweise zu Methanol und anschließend weiter zu Dimethylether umgesetzt. Die Gewinnung von Dimethylether aus Synthesegas ist thermodynamisch und wirtschaftlich vorteilhaft gegenüber einer Gewinnung aus Methanol.

Die vorliegende Erfindung betrifft die einstufige oder direkte Gewinnung von Dimethylether aus Synthesegas. Unter einer einstufigen oder direkten Gewinnung wird hier eine Gewinnung ohne eine zwischengeschaltete Methanolabtrennung verstanden, wie sie in einer zweistufigen Gewinnung erfolgt. Die ablaufenden Reaktionen können jedoch auch in einer einstufigen Gewinnung Methanol als Zwischenprodukt liefern, das jedoch in dem oder den verwendeten Reaktoren zumindest zum Teil weiter zu Dimethylether reagiert. Entsprechende Verfahren sind seit längerem bekannt und werden auch unten näher erläutert.

Die US 2013/0030063 A1 betrifft ein Verfahren zur Direktsynthese von Dimethylether aus Synthesegas in einem isotherm betriebenen Reaktor. Im Reaktorabstrom enthaltenes, nicht umgesetztes Synthesegas kann in den Reaktor zurückgeführt werden. Hierzu wird der Reaktorabstrom zunächst abgekühlt, so dass der überwiegende Anteil des Wassers und des Methanols sowie etwa 40% des Dimethylethers auskondensieren. Der Rest des Dimethylethers und der überwiegende Anteil des Kohlendioxids werden mittels eines Absorbers unter Verwendung von Methanol als Absorptionsmittel ausgewaschen. Dem Reaktor wird letztlich ein aufwendig an Kohlendioxid abgereicherter Recyclestrom und ein Frischeinsatz zugeführt, wobei eine Stöchiometriezahl des im Reaktor umgesetzten Gasgemischs maximal bei einem Wert von 2,05 liegt und sein Verhältnis von Kohlendioxid zu Kohlenmonoxid maximal 0,25 beträgt.

Bei der Herstellung von Dimethylether kann auch der in der EP 1 026 141 A1 behandelte Topsøe-Prozess zum Einsatz kommen. Dieser und andere Verfahren zur Herstellung von Dimethylether sind auch beispielsweise in dem Artikel "DME - the new wonder fuel?", Nitrogen & Methanol 260, 2002, Seiten 25 bis 31, erwähnt.

Es besteht weiterhin der Bedarf nach flexibleren und effizienteren Verfahren und Anlagen zur Gewinnung von Dimethylether aus Synthesegas.

### Offenbarung der Erfindung

Vor diesem Hintergrund schlägt die vorliegende Erfindung ein Verfahren und eine Anlage zur Gewinnung von Dimethylether aus Synthesegas gemäß den Merkmalen der unabhängigen Patentansprüche vor. Bevorzugte Ausführungsformen sind Gegenstand der Unteransprüche sowie der nachfolgenden Beschreibung.

Vor der Erläuterung der Merkmale und Vorteile der vorliegenden Erfindung werden deren Grundlagen und die verwendeten Begriffe erläutert.

Ist nachfolgend kurz von einer Gewinnung von Dimethylether die Rede, wird hierunter ein Verfahren verstanden, bei dem ein die bekannten Komponenten von Synthesegas enthaltender Einsatz, also ein Gasgemisch, das in geeigneten Anteilen zumindest Kohlenmonoxid, Kohlendioxid und Wasserstoff enthält, zu einem Dimethylether enthaltenden Produktstrom umgesetzt wird. Ein entsprechender Produktstrom enthält aufgrund der nicht vollständigen Reaktion und aufgrund des Auftretens von Nebenreaktionen bei der Synthese von Dimethylether, insbesondere in Abhängigkeit von den Charakteristika der verwendeten Katalysatoren und den jeweiligen Gehalten der Komponenten des Synthesegases, nicht ausschließlich Dimethylether sondern weitere Verbindungen. Diese sind zumindest Methanol, Wasser, Kohlendioxid, Kohlenmonoxid und Wasserstoff, jedoch auch geringere Mengen an Methan, Ethan, organischen Säuren und höheren Alkoholen. Diese weiteren Verbindungen müssen zumindest teilweise abgetrennt werden, um einerseits nachfolgende Trennschritte zu ermöglichen und andererseits Dimethylether in der geforderten Reinheit zu gewinnen.

Ein Fluid (die Bezeichnung Fluid wird nachfolgend auch kurz für entsprechende Ströme, Fraktionen usw. verwendet) ist von einem anderen Fluid (das hier auch als Ausgangsfluid bezeichnet wird) abgeleitet oder aus einem solchen Fluid gebildet, wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Strom kann auch beispielsweise einfach dadurch gebildet werden, dass er aus einem Speicherbehälter abgezogen wird.

Fluide können im hier verwendeten Sprachgebrauch reich oder arm an einer oder mehreren enthaltenen Komponenten sein, wobei reich für einen Gehalt von wenigstens 50%, 60%, 70%, 80% oder 90% und arm für einen Gehalt von höchstens 50%, 40%, 30%, 20% oder 10% auf molarer, Gewichts- oder Volumenbasis stehen kann. Sie können im hier verwendeten Sprachgebrauch angereichert oder abgereichert an einer oder mehreren Komponenten sein, wobei sich diese Begriffe auf einen entsprechenden Gehalt in einem Ausgangsfluid beziehen, aus dem das Fluid gebildet wurde. Das Fluid ist angereichert, wenn es zumindest den 1,1-fachen, 1,5-fachen, 2-fachen, 5-fachen, 10-fachen, 100-fachen oder 1.000-fachen Gehalt, abgereichert, wenn es höchstens den 0,9-fachen, 0,5-fachen, 0,1-fachen, 0,01-fachen oder 0,001-fachen Gehalt einer entsprechenden Komponente, bezogen auf das Ausgangsfluid, enthält. Ein überwiegend eine oder mehrere Komponenten enthaltendes Fluid enthält diese eine oder mehreren Komponenten zu wenigstens 50%, 60%, 70%, 80% oder 90% bzw. ist reich an diesen im Sinne der obigen Definition.

Nachfolgend werden zur Charakterisierung von Drücken und Temperaturen die Begriffe Druckniveau und Temperaturniveau verwendet, wodurch zum Ausdruck gebracht werden soll, dass Drücke und Temperaturen nicht in Form exakter Druck- bzw. Temperaturwerte verwendet werden müssen, um ein erfinderisches Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Unterschiedliche Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Dimethylether kann, wie eingangs bereits angesprochen, durch eine zweistufige Synthese aus Synthesegas über Methanol als Zwischenprodukt gewonnen werden. Entsprechende Verfahren sind beispielsweise ab Seite 171 im DME Handbook des Japan DME Forum, ISBN 978-4-9903839-0-9, 2007, beschrieben. Die zweistufige Gewinnung von Dimethylether aus Synthesegas zeichnet sich, wie erwähnt, dadurch aus, dass zunächst Methanol aus Synthesegas gewonnen wird, anschließend das nicht umgesetzte Synthesegas von den Kondensaten (Methanol und Wasser) abgetrennt wird, und das Methanol anschließend in einem weiteren Reaktor unter Gewinnung von Dimethylether und Wasser dehydratisiert wird.

Zur Gewinnung von Dimethylether werden normalerweise aufrechtstehende Rohrreaktoren eingesetzt, die jeweils von unten mit druckbeaufschlagtem, erhitztem Synthesegas beschickt werden. Ein erhaltener Produktstrom wird kopfseitig entnommen, gekühlt und einer Trennung zugeführt.

Die Gewinnung von Dimethylether in einem zweistufigen Verfahren ist teuer (und energetisch aufwendig), da hierzu eine vollständige Anlage zur Erzeugung von Methanol als Zwischenprodukt und zusätzlich Equipment für die Gewinnung von Dimethylether aus dem Methanol benötigt wird.

In der Patentliteratur wird bereits 1973 (DE 23 62 944 A1, US 4 098 809 A) die direkte oder einstufige Gewinnung von Dimethylether aus Synthesegas beschrieben. Diese zeichnet sich durch eine gemeinsame Reaktionsstufe aus, in der aus Wasserstoff, Kohlenmonoxid und Kohlendioxid gemeinsam Methanol und Dimethylether gewonnen werden. Hierauf aufbauend wurden in der Literatur weitere Verfahren beschrieben.

In einem bekannten Kombinationsverfahren, dem sogenannten Topsøe-Prozess, wie er im DME Handbook ab Seite 185, insbesondere auf Seite 187 beschrieben ist, wird ein dualer Katalysator, mit dem sich sowohl Methanol als auch Dimethylether bilden lassen, eingesetzt. Es kommen mindestens zwei Reaktoren ohne zwischengeschaltete Auftrennung zum Einsatz, wobei ein erster Reaktor isotherm gekühlt und ein zweiter Reaktor adiabat betrieben wird. Es wird ein Synthesegas mit einer Stöchiometriezahl (siehe unten) von ungefähr zwei eingesetzt. Es erfolgt eine parallele Produktion von Dimethylether und Methanol, wobei das Methanol in einem weiteren Reaktor nach Auftrennung der Komponenten zu Dimethylether umgesetzt werden kann. In dem Topsøe-Prozess sind immer mehrere Reaktoren (isotherm und adiabat betrieben) vorgesehen. Zusätzlich muss das in größeren Mengen anfallende Methanol in einem weiteren Reaktor zu Dimethylether umgesetzt werden, wenn keine gleichzeitige Gewinnung von Methanol erwünscht ist.

Die Direktsynthese von Dimethylether kann auch beispielsweise im Slurrybetrieb und bei vergleichsweise geringen Stöchiometriezahlen (siehe unten) erfolgen. Hierdurch kommt es jedoch immer zur Bildung von Kohlendioxid als Nebenprodukt, das von den jeweils nicht umgesetzten Verbindungen abgetrennt werden muss, um letztere als Recycle der Reaktion erneut zuführen zu können. Die genannten Reaktionen laufen hier nur bei niedrigem Kohlendioxidgehalt mit befriedigender Ausbeute ab.

### Vorteile der Erfindung

Die Erfindung schlägt ein Verfahren zur Gewinnung von Dimethylether aus Synthesegas vor, bei dem zumindest ein aus Synthesegas gebildeter Einsatzstrom zumindest einem Syntheseschritt unterworfen wird, bei welchem in dem Einsatzstrom enthaltene Komponenten teilweise zumindest zu Dimethylether umgesetzt werden. Hierbei wird zumindest ein Rohproduktstrom erhalten, der zumindest Dimethylether und nicht umgesetzte Komponenten des Einsatzstroms enthält.

Die Erfindung kommt also bei einer einstufigen Gewinnnung von Dimethylether zum Einsatz. Wie erwähnt, wird bei einer zweistufigen Gewinnnung von Dimethylether über das Zwischenprodukt Methanol letzteres abgetrennt und isoliert weiter zu Dimethylether umgesetzt. Es wird also nicht, wie im Rahmen der vorliegenden Erfindung der Fall, ein Rohproduktstrom erhalten, der zumindest Dimethylether und nicht umgesetzte Komponenten des Einsatzstroms enthält.

Ist hier davon die Rede, dass ein Einsatzstrom aus Synthesegas gebildet wird, umfasst dies insbesondere auch die Beimischung weiterer Komponenten zu einem Synthesegasstrom, wie bereits oben angegeben. Der Einsatzstrom selbst ist jener, der nach einer Beimischung dem wenigstens einen Syntheseschritt unterworfen wird.

Erfindungsgemäß ist vorgesehen, dass der Einsatzstrom zumindest Wasserstoff, Kohlenmonoxid und Kohlendioxid entsprechend einer Stöchiometriezahl von 2,0 bis 5,0 enthält, wobei der Kohlendioxidgehalt 4 bis 20 Molprozent beträgt, und dass der zumindest eine Syntheseschritt unter isothermen Bedingungen durchgeführt wird. Insbesondere kann die Synthese in einem einzigen isotherm betriebenen Reaktor ablaufen, es ist jedoch auch möglich, mehrere isotherm betriebene Reaktoren zu verwenden, die auf unterschiedlichen Temperaturniveaus arbeiten können.

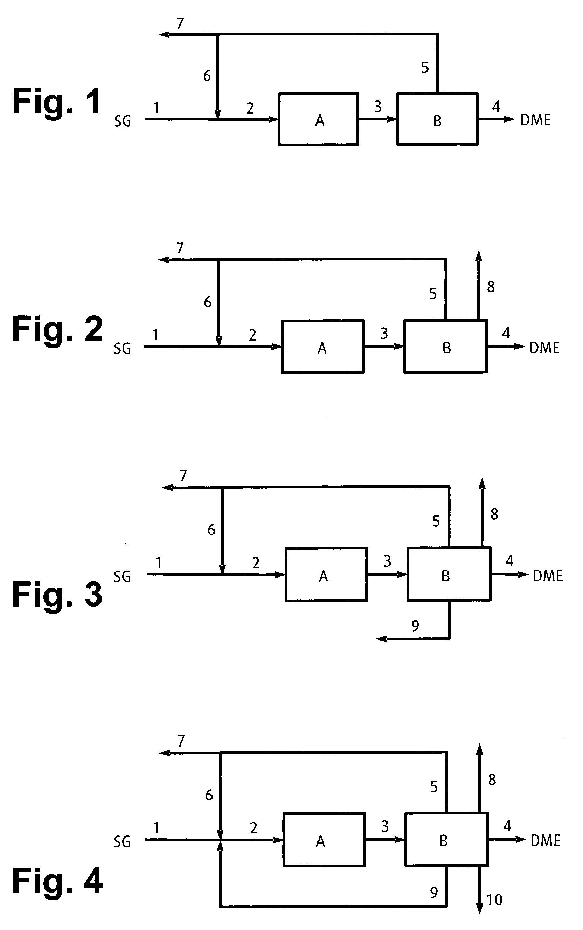

In der Regel wird der Einsatzstrom aus Synthesegas gebildet werden, dessen Stöchiometriezahl oberhalb von 2,0 liegt, beispielsweise bei 2,05. Der Einsatzstrom kann aber auch aus Synthesegas gebildet werden, dessen Stöchiometriezahl unterhalb von 2,0 liegt, beispielsweise bei 1,7. Dies kann beispielsweise bei der Beimischung eines Synthesegasstroms mit hoher Stöchiometriezahl erfolgen oder wenn ein in den Figuren 2 bis 4 dargestellter kohlendioxidreicher Strom 8 ausgeschleust wird. Der letztlich gebildete Einsatzstrom zeichnet sich jedoch erfindungsgemäß durch die genannte Stöchiometriezahl von 2,1 bis 5,0 aus. Alle Angaben bezüglich der erfindungsgemäß verwendeten Stöchiometriezahlen beziehen sich auf den Einsatzstrom, der tatsächlich dem zumindest einen Syntheseschritt unterworfen wird. Entsprechende Kohlendioxidgehalte stellen sich ein, wenn ein kohlendioxidhaltiger Recyclestrom zur Bildung des Einsatzstroms verwendet wird, auch wenn der verwendete Synthesegasstrom kohlendioxidarm ist.

Die Stöchiometriezahl beträgt hierbei insbesondere 2,1 bis 4,8, beispielsweise 2,2 bis 2,4, 2,4 bis 2,6, 2,6 bis 2,8, 2,8 bis 3,0, 3,0 bis 3,2, 3,2 bis 3,4, 3,4 bis 3,6, 3,6 bis 3,8, 3,8 bis 4,0, 4,0 bis 4,2, 4,2 bis 4,4, 4,4 bis 4,6 oder 4,6 bis 4,8.

Zur Charakterisierung des zur Gewinnung von Dimethylether eingesetzten Synthesegases oder auch aus Synthesegas und Recycleströmen gebildeter Einsatzströme wird häufig die genannte Stöchiometriezahl (engl. Stoichiometric Number) SN verwendet. Für diese gilt SN = (xH₂ - xCO₂) / (xCO + xCO₂), wobei x für den molaren Gehalt der Komponenten Wasserstoff (H₂), Kohlenmonoxid (CO) und Kohlendioxid (CO₂) steht. Die bei der herkömmlichen Gewinnung von Dimethylether direkt aus Synthesegas beobachteten Reaktionen lassen sich wie folgt angeben:

3 H₂ + 3 CO → CH₃OCH₃ + CO₂ (1)

4 H₂ + 2 CO → 2 CH₃OH → CH₃OCH₃ + H₂O (2)

Die in herkömmlichen Verfahren als ideal angesehene Stöchiometriezahl ergibt sich hieraus gemäß Reaktionsgleichung (1) zu

SN = (3 mol H₂ - 0 mol CO₂) / (0 mol CO + 3 mol CO₂) = 1,0

und gemäß Reaktionsgleichung (2) zu

SN = (4 mol H₂ - 0 mol CO₂) / (2 mol CO + 0 mol CO₂) = 2,0.

Bei der Reaktion gemäß Reaktionsgleichung (1) und mit SN = 1,0 kann sukzessive nahezu ein Vollumsatz der eingesetzten Komponenten erzielt werden. Allerdings muss das gebildete Kohlendioxid hierzu erneut durch einen Reformer geführt und dort zu Kohlenmonoxid umgewandelt werden. Dies ist ausgesprochen energieaufwendig. Kohlendioxid ist in herkömmlichen Verfahren, die bei entsprechend niedrigen Stöchiometriezahlen arbeiten, deshalb nicht erwünscht, weil es die beteiligten Reaktionen hemmen kann. Es muss daher aufwendig abgetrennt werden.

Hingegen ergibt sich bei einer Reaktion gemäß Reaktionsgleichung (2) und SN = 2,0 pro Durchlauf ein geringerer Umsatz und es bildet sich Wasser, in dem Wasserstoff und Sauerstoff gebunden sind, die gemäß Reaktionsgleichung (1) vollständig zu dem gewünschten Produkt umgesetzt werden können.

Wie erwähnt werden bei der Gewinnung von Dimethylether aus Synthesegas auch bei der Einhaltung der jeweils "idealen" Stöchiometriezahl die enthaltenen Komponenten niemals vollständig umgesetzt, zudem laufen die genannten Reaktionen, wenn auch in unterschiedlichen Anteilen, parallel zueinander ab. Damit findet sich im erhaltenen Rohprodukt, also am Ausgang des oder der verwendeten Reaktoren, immer auch Kohlendioxid, das sich insbesondere bei niedrigen Stöchiometriezahlen bildet.

Die vorliegende Erfindung beruht nun zumindest zum Teil auf der Erkenntnis, dass dieses Kohlendioxid bei höheren Stöchiometriezahlen zusammen mit den nicht umgesetzten Komponenten in den oder die verwendeten Reaktoren zurückgeführt werden kann, weil es ebenfalls umgesetzt werden kann. Es muss daher nicht aufwendig abgetrennt werden, wenn ein entsprechender Recyclestrom eingesetzt werden soll. Entsprechendes gilt auch für ein zur Bildung des Einsatzstroms verwendetes Synthesegas. Auch dieses weist immer eine bestimmte Menge an Kohlendioxid auf, das im Rahmen der vorliegenden Erfindung nicht oder zumindest nur in geringerem Umfang abgetrennt werden muss.

Im Rahmen der vorliegenden Erfindung hat sich dabei herausgestellt, dass zur Gewinnung von Dimethylether auch beispielsweise die nachfolgende Reaktion genutzt werden kann:

6 H₂ + 2 CO₂ → 2 CH₃OH + 2 H₂O → CH₃OCH₃ + 3 H₂O (3)

Die bei der Reaktion gemäß Gleichung (3) vorliegenden Stöchiometrieverhältnisse entsprechen einer Stöchiometriezahl von

SN = (6 mol H₂ - 2 mol CO₂) / (0 mol CO + 2 mol CO₂) = 2,0

und damit jener gemäß Reaktionsgleichung (2). Bei den nochmals höheren Stöchiometriezahlen, wie sie im Rahmen der vorliegenden Erfindung eingesetzt werden, ist jedoch ein deutlich höherer Umsatz von Kohlendioxid und Kohlenmonoxid zu Dimethylether zu beobachten.

Die hohen Stöchiometriezahlen der vorliegenden Erfindung werden beispielsweise durch einen Recycle der nicht umgesetzten Komponenten des Synthesegases in Kombination mit einem Makeup, also z.B. frischem Synthesegas, mit einer Stöchiometriezahl leicht oberhalb von zwei, beispielsweise bei SN = 2,05 erreicht. Durch den Recycle konzentriert sich überschüssiger Wasserstoff sukzessive auf. Die Stöchiometriezahl steigt damit an, auch wenn Kohlendioxid recycliert wird.

Die Vorteile der vorliegenden Erfindung ergeben sich insbesondere durch eine Kombination der vorgenannten Aspekte: Weil hier Kohlendioxid nicht in einem Reformer zu Kohlenmonoxid umgewandelt werden muss, ergibt sich ein Vorteil in der Gesamteffizienz des Verfahrens, wenngleich aufgrund der verwendeten höheren Stöchiometriezahlen gegebenenfalls geringere Umsätze pro Durchlauf erzielt werden. Letztere werden auch durch die nochmals höheren Stöchiometriezahlen kompensiert. Mit anderen Worten wird der Gesamtprozess deutlich effizienter, weil Kohlendioxid verwertet werden kann und nicht rezykliert werden muss.

Durch die erfindungsgemäße Verfahrensführung, in der vorzugsweise nur ein isotherm gekühlter Reaktor eingesetzt wird, ergeben sich ferner Vorteile gegenüber bekannten Verfahren wie dem erwähnten Topsøe-Prozess, in dem immer zwei unterschiedlich (isotherm und adiabat) betriebene Reaktoren verwendet werden.

Die isotherme Verfahrensführung ist auch deshalb vorteilhaft, weil bei der Kühlung des entsprechenden Reaktors Dampf produziert werden kann, der für andere Zwecke, beispielsweise für eine Feedvorwärmung, zur Verfügung steht.

Die Gehalte an Kohlendioxid und Kohlenmonoxid im Rahmen des erfindungsgemäßen Verfahrens können auch über das Verhältnis dieser Verbindungen zueinander angegeben werden. Das Kohlendioxid-/Kohlenmonoxidverhältnis liegt dabei erfindungsgemäß bei 0,5 bis 2, insbesondere 0,5 bis 1,0, 1,0 bis 1,5 oder 1,5 bis 2,0.

Im Rahmen der Erfindung kann also ein erhaltener Rohproduktstrom ohne Abtrennung von Kohlendioxid (jedoch nach Abtrennung der erwünschten Produkte, beispielsweise von Dimethylether) vollständig zur Reaktion zurückgeführt werden. Das erfindungsgemäße Verfahren erweist sich damit als einfacher in der Realisierung.

Erfindungsgemäß ist ferner, wie erwähnt, vorgesehen, lediglich isotherm betriebene Reaktoren zu verwenden. Hierdurch braucht nur ein einziger Reaktor bereitgestellt werden, die Verwendung unterschiedlich betriebener Reaktoren (isotherm und adiabat) ist hingegen nicht mehr erforderlich.

Das erfindungsgemäße Verfahren kann auch eine Synthese über Methanol als Zwischenprodukt umfassen, wobei letzteres jedoch nicht abgetrennt wird. Damit kann auf aufwendige Trenneinrichtungen verzichtet werden. Es wird also in dem wenigstens einen Syntheseschritt (beispielsweise in nur einem Reaktor) Wasserstoff und Kohlenmonoxid zunächst zu Methanol umgesetzt und das Methanol danach unter Anwesenheit der in dem Einsatzstrom enthaltenen Komponenten weiter zu Dimethylether umgesetzt. Im Rahmen des erfindungsgemäßen Verfahrens kann auch aus einem Rohproduktstrom abgetrenntes Methanol in den Syntheseschritt zurückgeführt werden. Hierdurch kann ein Reaktor, der herkömmlicherweise zur Dehydratisierung von Methanol zu Dimethylether eingesetzt wird, eingespart werden.

Wie erwähnt, werden vorteilhafterweise aus dem Rohproduktstrom die nicht umgesetzten Komponenten des Einsatzstroms zumindest teilweise abgetrennt und zurückgeführt. Sie können dabei zusammen mit dem Synthesegas zur Bildung des Einsatzstroms verwendet werden. Hierbei kann beispielsweise eine gemeinsame Verdichtung eines Synthesegasstroms und eines entsprechenden Recyclestroms erfolgen, wodurch auf getrennte Verdichterstufen verzichtet werden kann. Eine derartige gemeinsame Verdichtung liegt auch dann vor, wenn der Recyclestrom zwischen zwei Verdichterstufen eines Verdichters eingespeist wird, den der Synthesegasstrom vollständig durchläuft.

Insbesondere können aus dem Rohproduktstrom die nicht umgesetzten Komponenten des Einsatzstroms in einem überwiegend Wasserstoff, Kohlenmonoxid und Kohlendioxid enthaltenden Recyclestrom zumindest teilweise einem Synthesegasstrom beigemischt werden. Eine Abtrennung von Kohlendioxid ist, wie erwähnt, nicht oder nicht vollständig erforderlich, so dass ein erfindungsgemäßes Verfahren wirtschaftlich und energetisch günstig ist.

Es kann sich auch als vorteilhaft erweisen, aus dem Rohproduktstrom ferner einen Methanolstrom zu gewinnen und diesen zumindest teilweise zusammen mit dem Recyclestrom dem Synthesegasstrom beizumischen. Hierdurch kann in dem Methanolstrom enthaltenes Methanol parallel zur direkten Dimethylethergewinnung aus dem Synthesegas weiter zu Dimethylether umgesetzt werden.

Im Rahmen des erfindungsgemäßen Verfahrens wird der wenigstens eine Syntheseschritt vorteilhafterweise auf einem Temperaturniveau von 190 bis 310 °C und/oder auf einem Druckniveau von 20 bis 100 bar durchgeführt. Unter entsprechenden Bedingungen laufen die zuvor erläuterten Reaktionsschritte besonders effizient ab.

Im Rahmen des erfindungsgemäßen Verfahrens wird in dem wenigstens einen Syntheseschritt vorteilhafterweise zumindest ein Katalysator verwendet, der in der Lage ist, aus den genannten Ausgangsverbindungen über Methanol als Zwischenprodukt Dimethylether zu bilden, beispielsweise ein Kupfer-Zink-Katalysator. Dieser arbeitet auch unter den genannten Bedingungen effektiv.

Die Erfindung eignet sich insbesondere für Verfahren, bei denen aus dem Rohproduktstrom ferner Wasser, Dimethylether, Kohlendioxid und/oder Methanol abgetrennt werden. Die erhaltenen Komponenten können, je nach Bedarf, in einem verwendeten Verfahren eingesetzt werden, wie auch unten erläutert.

Eine Anlage zur Gewinnung von Dimethylether aus Synthesegas ist ebenfalls Gegenstand der vorliegenden Erfindung. Die Anlage weist wenigstens einen Dimethyletherreaktor auf, der dafür eingerichtet ist, zumindest einen aus Synthesegas gebildeten Einsatzstrom zumindest einem Syntheseschritt zu unterwerfen, bei welchem in dem Einsatzstrom enthaltene Komponenten teilweise zumindest zu Dimethylether umgesetzt werden. Es handelt sich also um wenigstens einen zur einstufigen Gewinnung von Dimethylether eingesetzten Dimethyletherreaktor. Sind mehrere Dimethyletherreaktoren vorhanden, können diese seriell oder parallel angeordnet und mit einem oder mehreren Einsatzströmen beschickt werden.

Bei der einstufigen Gewinnung von Dimethylether wird, wie bereits erläutert, zumindest ein Rohproduktstrom erhalten, der zumindest Dimethylether, Methanol und Wasser und die nicht umgesetzten Komponenten des Einsatzstroms enthält. Erfindungsgemäß zeichnet sich eine derartige Anlage durch Mittel aus, die dafür eingerichtet sind, den Einsatzstrom derart zu bilden, dass dieser zumindest Wasserstoff, Kohlenmonoxid und Kohlendioxid gemäß einer Stöchiometriezahl von 2,1 bis 5,0 aufweist und zumindest 4 bis 20 Molprozent Kohlendioxid enthält. Das Verhältnis von Kohlendioxid zu Kohlenmonoxid in dem Einsatzstrom liegt in einem Bereich von 0,5 bis 2. Ferner ist erfindungsgemäß wenigstens eine Kühleinrichtung vorgesehen, die dafür eingerichtet ist, den wenigstens einen Dimethyletherreaktor während des wenigstens einen Syntheseschritts isotherm zu betreiben. Wie erwähnt, laufen die Reaktionen zur Bildung von Dimethylether exotherm ab, so dass für einen ausschließlich isothermen Betrieb des oder der verwendeten Reaktoren eine entsprechende Wärmeabfuhr sichergestellt werden muss.

Eine derartige Anlage ist insbesondere dafür eingerichtet, ein Verfahren durchzuführen, wie es zuvor ausführlich erläutert wurde. Die erfindungsgemäße Anlage profitiert von den erläuterten Vorteilen, auf die daher ausdrücklich verwiesen wird.

Die Erfindung wird unter Bezugnahme auf die Zeichnungen näher erläutert, die Ausführungsformen der Erfindung zeigen.

### Kurze Beschreibung der Zeichnungen

Figur 1 zeigt ein Verfahren zur Gewinnung von Dimethylether aus Synthesegas gemäß einer Ausführungsform der Erfindung in schematischer Darstellung
Figur 2 zeigt ein Verfahren zur Gewinnung von Dimethylether aus Synthesegas gemäß einer Ausführungsform der Erfindung in schematischer Darstellung
Figur 3 zeigt ein Verfahren zur Gewinnung von Dimethylether aus Synthesegas gemäß einer Ausführungsform der Erfindung in schematischer Darstellung
Figur 4 zeigt ein Verfahren zur Gewinnung von Dimethylether aus Synthesegas gemäß einer Ausführungsform der Erfindung in schematischer Darstellung

### Ausführliche Beschreibung der Zeichnungen

In den Figuren sind einander entsprechende Elemente mit identischen Bezugszeichen angegeben und werden der Übersichtlichkeit halber nicht wiederholt erläutert.

Die Figuren 1 bis 4 zeigen Ausführungsformen eines erfindungsgemäßen Verfahrens zur Gewinnung von Dimethylether DME aus Synthesegas SG. Ein Syntheseschritt, der in einem oder mehreren isotherm geführten Reaktoren ablaufen kann, ist mit A und ein Abtrennschritt mit B bezeichnet. Allen dargestellten Ausführungsformen gemeinsam ist, dass ein Synthesegasstrom 1 nach Vereinigung mit zumindest einem weiteren Strom als Einsatzstrom 2 dem Syntheseschritt A unterworfen wird.

Der Synthesegasstrom 1 kann Synthesegas SG aus einem oder mehreren geeigneten vorgeschalteten Verfahrensschritten (beispielsweise aus Dampfreformierung, autothermer Reformierung, Trockenreformierung oder partieller Oxidation) enthalten. Der Synthesegasstrom 1 enthält Wasserstoff, Kohlenmonoxid und Kohlendioxid und typischerweise auch Nebenkomponenten wie Methan und Stickstoff.

Zur Bildung des Einsatzstroms 2 wird der Synthesegasstrom 1 (Makeupstrom) im Gegensatz zu herkömmlichen Verfahren zur einstufigen Gewinnung von Dimethylether aus Synthesegas nicht oder nur teilweise von Kohlendioxid befreit. Zur Bildung des Einsatzstroms 2 wird der Synthesegasstrom 1 ferner mit zumindest einem Recyclestrom 6, der aus in dem Abtrennschritt B gewonnenen Komponenten gebildet wird, gemischt. Der Recyclestrom 6 kann entweder in einem Recycleverdichter verdichtet werden, so dass der Synthesegasstrom 1 und der Recyclestrom 6 auf dem gleichen Druckniveau vorliegen, oder er wird gemeinsam mit dem Synthesegasstrom 1 verdichtet. Der Recyclestrom 6 enthält dabei zumindest einige der in dem Syntheseschritt A nicht umgesetzten Komponenten des Synthesegasstroms 1 bzw. des Einsatzstroms 2. Der Einsatzstrom 2 zeichnet sich in der dargestellten Ausführungsform der Erfindung gegenüber dem Stand der Technik durch eine vergleichsweise hohe Stöchiometriezahl und einen vergleichsweise hohen Kohlendioxidgehalt aus, wie zuvor angegeben.

In dem Syntheseschritt A wird aus dem Einsatzstrom 2 ein Dimethylether enthaltender Rohproduktstrom 3 gewonnen. Der Rohproduktstrom 3 kann neben Dimethylether auch nicht umgesetztes Synthesegas, Methanol, Wasser und ggf. (zumindest in dem Syntheseschritt A) inerte Gase enthalten. Er wird dem Abtrennschritt B unterworfen, in welchem zumindest ein überwiegend Dimethylether enthaltender Produktstrom 4 gewonnen wird. Der Produktstrom 4 kann neben Dimethylether auch größere Mengen an Methanol und Wasser sowie Verunreinigungen wie Kohlendioxid und Alkane enthalten. Die erzeugte Reinheit richtet sich nach Wirtschaftlichkeitserwägungen.

In der in Figur 1 gezeigten Ausführungsform der Erfindung wird neben dem Produktstrom 4 ein Abstrom 5 aus nicht umgesetztem Synthesegas SG bzw. den nicht umgesetzten Komponenten des Synthesegasstroms 1 bzw. des Einsatzstroms 2 erhalten. Der Abstrom 5 enthält überwiegend Wasserstoff, Kohlenmonoxid, Kohlendioxid, Methan und weitere leichte inerte Gase. Der Abstrom 5 wird unter Erhalt des Recyclestroms 6 und eines Reststroms 7 aufgeteilt, wobei der Reststrom 7 üblicherweise aus nur 1 bis 10% des Abstroms 5 gebildet wird. Der Reststrom 7 kann als Brenngas, z.B. im Brenner eines Reformers zur Herstellung des Synthesegases SG, als Einsatz in einem solchen Reformer, zur Erzeugung eines wasserstoffreichen Stroms, z.B. durch Druckwechselabsorption, als Produktexport und/oder in anderen Anlagenteilen, beispielsweise zur Erdgasentschwefelung stromauf eines Reformers, eingesetzt werden.

In Figur 2 ist eine weitere Ausführungsform der Erfindung dargestellt, bei der ein kohlendioxidreicher Strom 8 in dem Trennschritt B anfällt. Das in diesem kohlendioxidreichen Strom 8 beispielsweise zu mindestens 80% enthaltene Kohlendioxid findet sich gemäß Figur 1 in dem Abstrom 5 wieder. Das Kohlendioxid kann damit je nach Ausgestaltung der Trenneinheit B entweder zusammen mit weiteren Komponenten (Figur 1) oder als separater Strom 8 (Figuren 2 bis 4) erhalten werden und dabei in gasförmigem oder flüssigem Zustand vorliegen. Der Strom 8 kann beispielsweise mit dem Abstrom 5 (ggf. nach Druckanhebung) gemischt, zur Erzeugung des Synthesegases SG als Einsatz rezykliert, vor oder während einer Verdichtung mit dem Synthesegasstrom 1 gemischt und/oder, z.B. im Brenner eines Reformers zur Herstellung des Synthesegases SG, verwendet werden. Die Bereitstellung eines separaten Stroms 8 erhöht damit die Flexibilität.

In Figur 3 ist eine weitere Ausführungsform der Erfindung dargestellt, bei der ein methanol- und/oder wasserreicher Strom 9 in dem Trennschritt B anfällt. In dieser Ausführungsform der Erfindung kann der Produktstrom 4 besonders reich an Dimethylether und arm an Methanol und/oder Wasser sein. Der methanol- und/oder wasserreiche Strom 9 kann zur Gewinnung des Synthesegases SG rezykliert werden.

In Figur 4 ist eine weitere Ausführungsform der Erfindung dargestellt, bei der ein methanolreicher Strom 9 und ein wasserreicher Strom 10 in dem Trennschritt B getrennt anfallen. Der methanolreiche Strom 9 kann exportiert oder zur Gewinnung des Synthesegases SG rezykliert werden. Der methanolreiche Strom 9 kann auch in den Syntheseschritt A zurückgeführt und zur Bildung von Dimethylether verwendet werden. Es ist kein weiterer Reaktor zur Dehydratisierung des Methanols erforderlich. Der wasserreiche Strom 10 kann einer Abwasserbehandlung unterworfen werden.

## Patentansprüche

1. Verfahren zur Gewinnung von Dimethylether (DME) aus Synthesegas (SG), bei dem zumindest ein aus Synthesegas (SG) gebildeter Einsatzstrom (2) zumindest einem Syntheseschritt (A) unterworfen wird, in welchem in dem Einsatzstrom (2) enthaltene Komponenten teilweise zumindest zu Dimethylether (DME) umgesetzt werden, wobei zumindest ein Rohproduktstrom (3) erhalten wird, der zumindest Dimethylether (DME) und nicht umgesetzte Komponenten des Einsatzstroms (2) enthält, **dadurch gekennzeichnet, dass** der Einsatzstrom (2) Wasserstoff, Kohlenmonoxid und Kohlendioxid entsprechend einer Stöchiometriezahl von 2,1 bis 5,0 und 4 bis 20 Molprozent Kohlendioxid enthält und das Verhältnis von Kohlendioxid zu Kohlenmonoxid in dem Einsatzstrom (2) in einem Bereich von 0,5 bis 2 liegt, und dass der wenigstens eine Syntheseschritt (A) unter isothermen Bedingungen durchgeführt wird.

2. Verfahren nach Anspruch 1, bei dem in dem wenigstens einen Syntheseschritt (A) Wasserstoff, Kohlenmonoxid und/oder Kohlendioxid zu Methanol umgesetzt werden und das Methanol unter Anwesenheit anderer in dem Einsatzstrom (2) enthaltener Komponenten weiter zu Dimethylether umgesetzt wird.

3. Verfahren nach Anspruch 1 oder 2, bei dem aus dem Rohproduktstrom (3) die nicht umgesetzten Komponenten des Einsatzstroms (2) zumindest teilweise abgetrennt und zusammen mit dem Synthesegas (SG) zur Bildung des Einsatzstroms (2) verwendet werden.

4. Verfahren nach Anspruch 3, bei dem aus dem Rohproduktstrom (3) die nicht umgesetzten Komponenten des Einsatzstroms (2) in Form eines überwiegend Wasserstoff, Kohlenmonoxid und Kohlendioxid enthaltenden Recyclestroms (6) zumindest teilweise einem Synthesegasstrom (1) beigemischt werden.

5. Verfahren nach Anspruch 4, bei dem aus dem Rohproduktstrom (3) ferner ein Methanolstrom (9) gewonnen und zumindest teilweise zusammen mit dem Recyclestrom (6) dem Synthesegasstrom (1) beigemischt wird.

6. Verfahren nach einem der vorstehenden Ansprüche, bei dem der wenigstens eine Syntheseschritt (A) auf einem Temperaturniveau von 190 bis 310 °C und/oder auf einem Druckniveau von 20 bis 100 bar durchgeführt wird.

7. Verfahren nach einem der vorstehenden Ansprüche, bei dem in dem wenigstens einen Syntheseschritt (A) zumindest ein Katalysator verwendet wird, der in der Lage ist, sowohl aus Wasserstoff und Kohlenmonoxid als auch aus Wasserstoff und Kohlendioxid über Methanol als Zwischenprodukt Dimethylether zu bilden.

8. Verfahren nach einem der vorstehenden Ansprüche, bei dem aus dem Rohproduktstrom (3) ferner Wasser, Dimethylether, Kohlendioxid und/oder Methanol zumindest teilweise abgetrennt werden.

9. Anlage zur Gewinnung von Dimethylether (DME) aus Synthesegas (SG), die wenigstens einen Dimethyletherreaktor aufweist, der dafür eingerichtet ist, zumindest einen aus Synthesegas (SG) gebildeten Einsatzstrom (2) zumindest einem Syntheseschritt (A) zu unterwerfen, bei welchem in dem Einsatzstrom (2) enthaltene Komponenten teilweise zumindest zu Dimethylether (DME) umgesetzt werden, wodurch zumindest ein Rohproduktstrom (3) erhalten wird, der zumindest Dimethylether (DME) und nicht umgesetzte Komponenten des Einsatzstroms (2) enthält, **gekennzeichnet durch** Mittel, die dafür eingerichtet sind, den Einsatzstrom (2) derart zu bilden, dass dieser Wasserstoff, Kohlenmonoxid und Kohlendioxid entsprechend einer Stöchiometriezahl von 2,1 bis 5,0 und 4 bis 20 Molprozent Kohlendioxid enthält und das Verhältnis von Kohlendioxid zu Kohlenmonoxid in dem Einsatzstrom (2) in einem Bereich von 0,5 bis 2 liegt, und wenigstens eine Kühleinrichtung, die dafür eingerichtet ist, den wenigstens einen Dimethyletherreaktor während des wenigstens einen Syntheseschritts (A) isotherm zu betreiben.

10. Anlage nach Anspruch 9, die zur Durchführung eines Verfahrens nach einem der Ansprüche 1 bis 8 eingerichtet ist.

## Claims

1. Process for production of dimethyl ether (DME) from synthesis gas (SG), in which at least one feed stream (2) formed from synthesis gas (SG) is subjected to at least one synthesis step (A), in which components present in the feed stream (2) are at least in part converted to dimethyl ether (DME), wherein at least one crude product stream (3) is obtained which contains at least dimethyl ether (DME) and unreacted components of the feed stream (2), **characterized in that** the feed stream (2) contains hydrogen, carbon monoxide and carbon dioxide corresponding to a stoichiometric number of 2.1 to 5.0 and contains 4 to 20 mol percent carbon dioxide, and the ratio of carbon dioxide to carbon monoxide in the feed stream (2) is in a range from 0.5 to 2, and **in that** the at least one synthesis step (A) is carried out under isothermal conditions.

2. Process according to Claim 1, in which hydrogen, carbon monoxide and/or carbon dioxide are converted to methanol in the at least one synthesis step (A) and the methanol is further converted to dimethyl ether in the presence of other components present in the feed stream (2).

3. Process according to Claim 1 or 2, in which, from the crude product stream (3), the unreacted components of the feed stream (2) are at least in part separated off and are used together with the synthesis gas (SG) to form the feed stream (2).

4. Process according to Claim 3, in which, from the crude product stream (3), the unreacted components of the feed stream (2) are added in the form of a recycle stream (6) predominantly containing hydrogen, carbon monoxide and carbon dioxide at least in part to a synthesis gas stream (1).

5. Process according to Claim 4, in which, from the crude product stream (3), in addition a methanol stream (9) is produced and is added at least in part together with the recycle stream (6) to the synthesis gas stream (1).

6. Process according to any one of the preceding claims, in which the at least one synthesis step (A) is carried out at a temperature level of 190 to 310°C and/or at a pressure level of 20 to 100 bar.

7. Process according to any one of the preceding claims, in which, in the at least one synthesis step (A), at least one catalyst is used which is able to form dimethyl ether, not only from hydrogen and carbon monoxide, but also from hydrogen and carbon dioxide via methanol as intermediate.

8. Process according to any one of the preceding claims, in which, from the crude product stream (3), in addition water, dimethyl ether, carbon dioxide and/or methanol are at least in part separated off.

9. System for production of dimethyl ether (DME) from synthesis gas (SG) which has at least one dimethyl ether reactor which is equipped to subject at least one feed stream (2) formed from synthesis gas (SG) to at least one synthesis step (A) in which components that are present in the feed stream (2) are at least in part converted to dimethyl ether (DME), as a result of which at least one crude product stream (3) is obtained which contains at least dimethyl ether (DME) and unreacted components of the feed stream (2), **characterized by** means which are equipped to form the feed stream (2) in such a manner that the latter contains hydrogen, carbon monoxide and carbon dioxide corresponding to a stoichiometric number of 2.1 to 5.0 and contains 4 to 20 mol percent carbon dioxide, and the ratio of carbon dioxide to carbon monoxide in the feed stream (2) is in a range from 0.5 to 2, and at least one cooling appliance which is equipped to operate the at least one dimethyl ether reactor isothermally during the at least one synthesis step (A).

10. System according to Claim 9, which is equipped for carrying out a process according to any one of Claims 1 to 8.

## Revendications

1. Procédé pour la préparation de diméthyléther (DME) à partir de gaz de synthèse (GS), dans lequel au moins un flux d'utilisation (2) formé de gaz de synthèse (GS) est soumis à au moins une étape de synthèse (A), dans laquelle des composants contenus dans le flux d'utilisation (2) sont transformés au moins partiellement en diméthyléther (DME), au moins un flux de produit brut (3) étant obtenu, qui contient au moins du diméthyléther (DME) et des composants non transformés du flux d'utilisation (2), **caractérisé en ce que** le flux d'utilisation (2) contient de l'hydrogène, du monoxyde de carbone et du dioxyde de carbone, conformément à un indice de stoechiométrie de 2,1 à 5,0, et 4 à 20% en poids de dioxyde de carbone et le rapport de dioxyde de carbone à monoxyde de carbone dans le flux d'utilisation (2) se situe dans une plage de 0,5 à 2 et **en ce que** ladite au moins une étape de synthèse (A) est réalisée dans des conditions isothermiques.

2. Procédé selon la revendication 1, dans lequel, dans ladite au moins une étape de synthèse (A), on transforme de l'hydrogène, du monoxyde de carbone et/ou du dioxyde de carbone en méthanol et le méthanol est transformé davantage, en présence d'autres composants contenus dans le flux d'utilisation (2), en diméthyléther.

3. Procédé selon la revendication 1 ou 2, dans lequel on sépare au moins partiellement du flux de produit brut (3) les composants non transformés du flux d'utilisation (2) et on les utilise ensemble avec le gaz de synthèse (GS) pour la formation du flux d'utilisation (2).

4. Procédé selon la revendication 3, dans lequel les composants non transformés du flux d'utilisation (2) provenant du flux de produit brut (3) sont mélangés au moins partiellement à un flux de gaz de synthèse (1) sous forme d'un flux de recyclage (6) contenant essentiellement de l'hydrogène, du monoxyde de carbone et du dioxyde de carbone.

5. Procédé selon la revendication 4, dans lequel un flux de méthanol (9) est en outre obtenu à partir du flux de produit brut (3) et mélangé au moins partiellement ensemble avec le flux de recyclage (6) au flux de gaz de synthèse (1).

6. Procédé selon l'une quelconque des revendications précédentes, dans lequel ladite au moins une étape de synthèse (A) est réalisée à un niveau de température de 190 à 310°C et/ou à un niveau de pression de 20 à 100 bars.

7. Procédé selon l'une quelconque des revendications précédentes, dans lequel on utilise, dans ladite au moins une étape de synthèse (A), au moins un catalyseur qui est en mesure de former du diméthyléther, tant à partir d'hydrogène et de monoxyde de carbone qu'à partir d'hydrogène et de dioxyde de carbone, via le méthanol comme produit intermédiaire.

8. Procédé selon l'une quelconque des revendications précédentes, dans lequel on sépare en outre au moins partiellement, du flux de produit brut, (3), de l'eau, du diméthyléther, du dioxyde de carbone et/ou du méthanol.

9. Installation pour la production de diméthyléther (DME) à partir de gaz de synthèse (GS), qui présente au moins un réacteur pour le diméthyléther qui est conçu pour soumettre au moins un flux d'utilisation (2) formé de gaz de synthèse (GS) à au moins une étape de synthèse (A), dans laquelle des composants contenus dans le flux d'utilisation (2) sont transformés au moins partiellement en diméthyléther (DME), suite à quoi au moins un flux de produit brut (3) est obtenu, qui contient au moins du diméthyléther (DME) et des composants non transformés du flux d'utilisation (2), **caractérisé par** des moyens qui sont conçus pour former le flux d'utilisation (2) de manière telle que celui-ci contient de l'hydrogène, du monoxyde de carbone et du dioxyde de carbone, conformément à un indice de stoechiométrie de 2,1 à 5,0, et 4 à 20% en mole de dioxyde de carbone et le rapport de dioxyde de carbone à monoxyde de carbone dans le flux d'utilisation (2) se situe dans une plage de 0,5 à 2, et au moins un dispositif de refroidissement qui est conçu pour faire fonctionner ledit au moins un réacteur pour le diméthyléther de manière isothermique pendant ladite au moins une étape de synthèse (A).

10. Installation selon la revendication 9, qui est conçue pour réaliser un procédé selon l'une quelconque des revendications 1 à 8.
